(19) Europäisches Patentamt
European Patent Office
Office européen des brevets

(11) Numéro de publication : **0 349 380 B1**

# (12) FASCICULE DE BREVET EUROPEEN

(45) Date de publication du fascicule du brevet : **26.08.92 Bulletin 92/35**

(51) Int. Cl.$^5$ : **C07C 323/52**

(21) Numéro de dépôt : **89401737.5**

(22) Date de dépôt : **20.06.89**

## (54) Nouveaux esters renfermant un groupement dithioacetal, leur préparation et leur application à la stabilisation des matières organiques.

(30) Priorité : **29.06.88 FR 8808762**

(43) Date de publication de la demande : **03.01.90 Bulletin 90/01**

(45) Mention de la délivrance du brevet : **26.08.92 Bulletin 92/35**

(84) Etats contractants désignés : **AT BE CH DE FR GB IT LI LU NL SE**

(56) Documents cités : **US-A- 3 743 623**

(73) Titulaire : **M & T CHEMICALS, INC.**
**One Woodbridge Center**
**Woodbridge, New Jersey 07095 (US)**

(72) Inventeur : **Tozzolino, Pierre**
**Chemin Carrérot**
**F-64160 Serres-Morlaas (FR)**
Inventeur : **Ranceze, Dominique**
**Lotissement Tauzier Mazerolles**
**F-64230 Lescar (FR)**

(74) Mandataire : **Rochet, Michel et al**
**ELF ATOCHEM S.A. Département Propriété Industrielle 4-8, Cours Michelet La Défense 10 - Cedex 42**
**F-92091 Paris-La-Défense (FR)**

**EP 0 349 380 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des composés organiques soufrés qui renferment à la fois des groupes esters et un groupe dithioacétal, leur préparation ainsi que leur application à la stabilisation des matières organiques.

Les thioéthers portant des fonctions esters sont connus comme stabilisants des matières organiques.

Ainsi par exemple, des thioétheresters de formule :

$S\,(CH_2)_2\,COOR_2$, dans laquelle R représente $C_{18}H_{37}$- ou $C_{12}H_{25}$-, sont utilisés pour assurer une bonne protection des matières plastiques contre l'oxydation (Information chimie N° 265, octobre 1985, page 173).

Le brevet US 2 393 327 décrit des composés qui, ayant deux atomes de soufre séparés par au moins deux atomes de carbone dans la chaîne principale, sont utilisés comme platifiants des résines synthétiques.

Dans le brevet US 2 530 882, on préconise comme plastifiants des résines vinyliques des dithiodialkylesters.

Le brevet US 3 494 947 décrit des dithiodiabiétylesters ayant de 1 à 5 atomes de carbone entre les atomes de soufre.

Ces composés sont associés avec.des phénols encombrés pour stabiliser les polyoléfines contre la thermooxydation.

Les brevets US 3 637 809 et US 3 743 623 décrivent des dithiotriesters utilisés comme antioxydants des résines synthétiques. Dans ces composés, les atomes de soufre sont séparés par une chaîne alkyle de 2 à 10 atomes de carbone. Les fonctions esters renferment des phénols encombrés.

Il a maintenant été trouvé des composés organiques soufrés qui renferment à la fois des groupes esters et un groupement dithioacétal répondant à la formule générale :

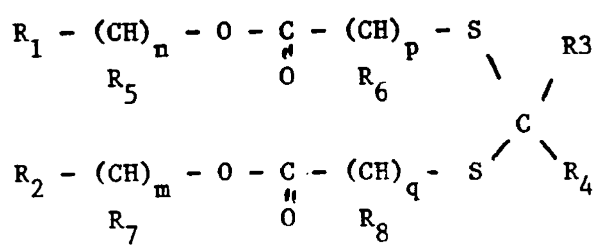

(I)

dans laquelle, $R_1$ et $R_2$, identiques ou différents, représentent des radicaux aryles, arylthio, alklthioaryles dans lesquels le groupement aryle peut être un radical phényle substitué ou non par des hydroxyles, et/ou par des radicaux hydrocarbonés aliphatiques linéaires ou ramifiés et ayant jusqu'à 6 atomes de carbone ; $R_3$ et $R_4$, identiques ou différents, représentent des atomes d'hydrogène, des radicaux hydrocarbonés aliphatiques linéaires ou ramifiés et ayant jusqu'à 6 atomes de carbone, des radicaux cycloaliphatiques, des radicaux phényles substitués ou non par des restes hydrocarbonés aliphatiques linéaires ou ramifiés et ayant jusqu'à 6 atomes de carbone ;

$R_3$ et $R_4$ pouvant également former ensemble avec le radical méthylène et éventuellement un hétéroatome un radical cyclique ou hétérocyclique ayant de 5 à 12 atomes de carbone ;

$R_5$, $R_5$, $R_7$ et $R_8$, identiques ou différents, représentent des atomes d'hydrogène, des radicaux hydrocarbonés aliphatiques linéaires ou ramifiés et ayant jusqu'à 6 atomes de carbone, des radicaux cycloaliphatiques, des radicaux phényles ;

n, m, p et q sont des nombres entiers, n et m pouvant être compris entre 1 et 12, p et q pouvant être compris entre 1 et 10.

Parmi les composés de formule I, on préfère ceux dans lesquels $R_5$, $R_6$, $R_7$ et $R_8$ représentent des atomes d'hydrogène, $R_3$ et $R_4$ représentent des atomes d'hydrogène, des radicaux hydrocarbonés aliphatiques linéaires ayant de 1 à 6 atomes de carbone, des radicaux phényles ou bien forment ensemble avec le radical méthylène, un groupe cycloalkylène ayant de 5 à 12 atomes de carbone, et $R_1$ et $R_2$ sont des hydroxyphényles substitués dans lesquels les groupements hydroxyles sont encombrés par 1 ou 2 restes hydrocarbonés aliphatiques ramifiés ayant de 3 à 6 atomes de carbone.

Parmi les produits de formule I, on citera tout particulièrement les produits dans la formule desquels $R_1$ et $R_2$, identiques ou différents, représentent l'un des groupements :

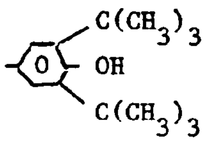
,
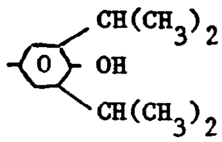
et
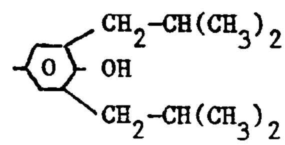

et n, m, p et q sont compris entre 1 et 4.

Parmi les composés soufrés qui renferment des groupes esters et un groupe dithioacétal qui répondent à la formule I, on peut citer notamment les composés suivants :

- bis{[(ditert.butyl-3,5 hydroxy-4 phényl)-3 propoxycarbonyl] -2 éthylthio} méthane.
- bis{[(ditert.butyl-3,5 hydroxy-4 phényl)-2 éthoxycarbonyl] -2 éthylthio} méthane.
- bis[(ditert.butyl-3,5 hydroxy-4 phényl)-3 propoxycarbonylméthylthio] méthane.
- bis[(ditert.butyl-3,5 hydroxy-4 phényl)-2 éthoxycarbonylméthylthio] méthane.
- α,α-bis{[(ditert.butyl-3,5 hydroxy-4 phényl)-3 propoxycarbonyl] -2 éthylthio} toluène.
- α,α-bis{[(ditert.butyl-3,5 hydroxy-4 phényl)-2 éthoxycarbonyl -2 éthylthio} toluène.
- α,α-bis [(ditert.butyl-3,5 hydroxy-4 phényl)-3 propoxycarbonylméthylthio] toluène.
- α,α-bis [(ditert.butyl-3,5 hydroxy-4 phényl)-2 éthoxycarbonylméthylthio] toluène.
- bis{[(ditert.butyl-3,5 hydroxy-4 phényl)-3 propoxycarbonyl] -2 éthylthio} -1,1 cyclohexane.
- bis {[(ditert.butyl-3,5 hydroxy-4 phényl)-2 éthoxycarbonyl] -2 éthylthio} -1,1 cyclohexane.
- bis [(ditert.butyl-3,5 hydroxy-4 phényl)-3 propoxycarbonylméthylthio] -1,1 cyclohexane.
- bis [(ditert.butyl-3,5 hydroxy-4 phényl)-2 éthoxycarbonylméthylthio] -1,1 cyclohexane.
- bis{[(ditert.butyl-3,5 hydroxy-4 phényl)-3 propoxycarbonyl] -2 éthylthio} -2,2 propane.
- bis{[(ditert.butyl-3,5 hydroxy-4 phényl)-2 éthoxycarbonyl] -2 éthylthio} -2,2 propane.
- bis [(ditert.butyl-3,5 hydroxy-4 phényl)-3 propoxycarbonylméthylthio] -2,2 propane.
- bis [(ditert.butyl-3,5 hydroxy-4 phényl)-2 éthoxycarbonylméthylthio] -2,2 propane.

Les produits conforment à l'invention peuvent être préparés par réaction d'un phénol-alcool, d'un mercaptoacide et d'un aldéhyde ou d'une cétone. Selon une première variante, on peut dans un premier temps faire réagir un phénol-alcool avec un mercapto-acide, en présence d'un catalyseur acide puis ensuite à condenser le mercapto-ester (1) obtenu avec un aldéhyde ou une cétone selon les réactions :

a) 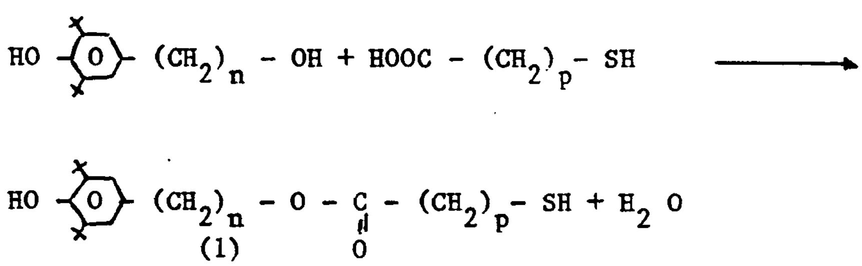

b) 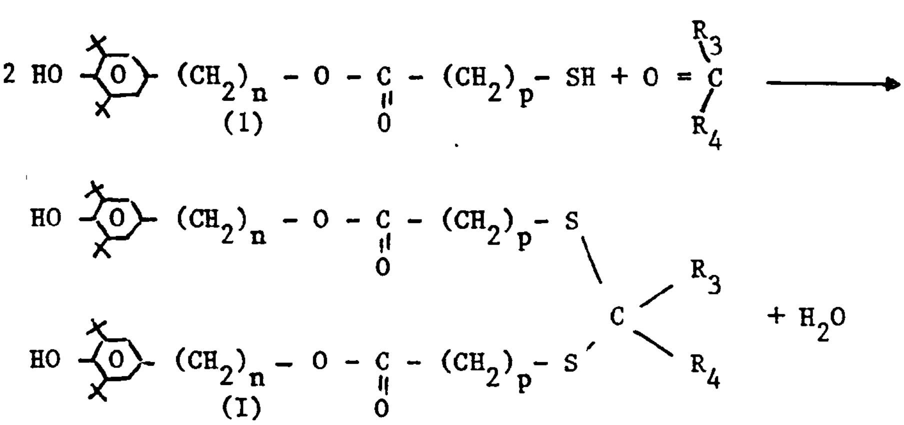

selon une autre variante on peut condenser un aldéhyde ou une cétone avec un mercaptoacide puis faire réagir le dithio-acétal diacide (2) obtenu avec un phénol-alcool en présence d'un catalyseur acide selon les réactions :

c) 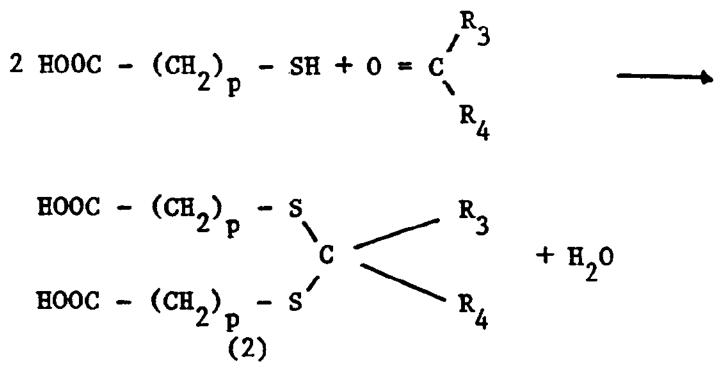

(2)

d) 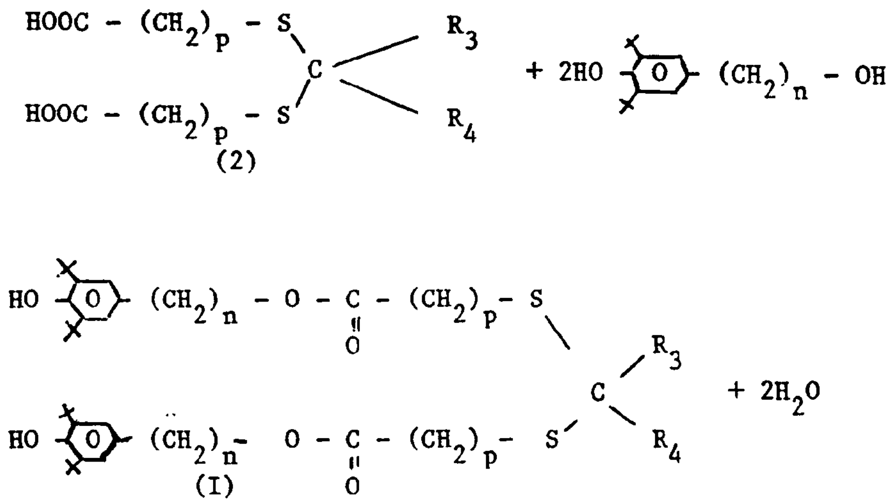

(2)

(I)

Conformément à l'invention les réactions c) et d) peuvent être réalisées simultanément.

Dans les deux cas les réactions ont lieu avantageusement dans un solvant inerte vis-à-vis des réactifs et sont généralement réalisées à une température comprise entre 50°C et 170°C, de préférence entre 80°C et 130°C pendant une durée qui peut aller de une à plusieurs heures sous bonne agitation.

Dans le procédé selon l'invention, on peut utiliser dans les réactions b) ou c) des phénols-alcools ou des thioacides identiques ou différents. On opère de préférence en utilisant des quantités stoéchiométriques des réactifs mis en jeu, mais il est possible d'utiliser un léger excès d'aldéhyde ou de cétone par rapport au mercaptoester (réaction b) ou au thioacide (réaction c).

Comme solvants inertes, on peut citer à titre non limitatif les hydrocarbures aliphatiques, cycloaliphatiques ou aromatiques tels que heptane, cyclohéxane, benzène, toluène, xylènes, solvants naphta et similaires, les hydrocarbures halogènes, comme le chloroforme et le tétrachlorure de carbone, les éthers tels que le tétrahydrofuranne ou le dioxanne.

Comme catalyseurs acides convenant particulièrement bien à la formation du mercapo-ester (1), du dithioacétal acide et du dithioacétal ester selon le procédé de l'invention on peut citer l'acide sulfurique, l'acide paratoluènesulfonique.

La quantité de catalyseur peut varier dans les limites comprises entre 0,1 et 2 % et de préférence entre 0,5 et 1 % en poids par rapport aux réactifs mis en oeuvre.

Dans le procédé selon l'invention les aldéhydes et les cétones que l'on peut utiliser sont par exemple, donnés à titre non limitatif, le formaldéhyde, tel quel ou sous forme de polyoxyméthylène ou de trioxyméthylène, l'acétaldéhyde, le benzaldéhyde, l'acétone, la cyclohéxanone, la diphénylcétone ...

Parmi les mercapto-acides que l'on peut utiliser selon l'invention on peut citer à titre non limitatif l'acide mercapto-3 propanoïque, l'acide thioglycolique...

De même parmi les phénols-alcools que l'on peut utiliser selon l'invention, on peut citer à titre non limitatif le (ditert.butyl-3,5 hydroxy-4 phényl)-2 hydroxy-1 éthane, le (ditert.butyl-3,5 hydroxy-4 phényl)-3 hydroxy-1 propane...

Les nouveaux composés répondant à la formule I conviennent particulièrement bien pour stabiliser les matières organiques et notamment les résines synthétiques contre la dégradation thermoxydative qui se produit aussi bien au moment de leur transformation que durant leur existence. Ces composés confèrent aux résines synthétiques une résistance améliorée contre les effets dégradants de la chaleur et de l'oxydation qui se

produisent lors de la mise en oeuvre des polymères. Même à température élevée et pendant des durées de contact relativement longues lors de la transformation à l'état fondu des résines synthétiques, ces nouveaux antioxydants confèrent aux résines une bonne stabilité sans modification de couleur.

Lorsque les composés selon l'invention sont utilisés comme antioxydants pour stabiliser les matières organiques et, plus précisément pour stabiliser les résines synthétiques, on les incorpore avantageusement entre 0,005 et 5 % en poids par rapport à la matière à stabiliser, de préférence 0,01 à 1 % et plus préférentiellement 0,02 à 0,5 %.

Les composés selon l'invention peuvent être utilisés pour stabiliser contre la dégradation thermooxydative toutes matières organiques et notamment, toutes résines synthétiques parmi lesquelles on peut citer, à titre non limitatif :

Les polyoléfines, les polymères vinyliques ou vinyliques halogénés, les résines acryliques, méthacryliques, les époxydes, les polyacétals, les polyphénoxydes, les polycarbonates, les polysulfones, les polyuréthannes, les polyamides, les polyesters, les phénoplastes, les aminoplastes, les résines glycérophtaliques, les résines cellulosiques, les caoutchoucs, les protéines et autres composés naturels, notamment les huiles, les graisses, etc.

Les exemples suivants explicitent l'invention, sans la limiter.

EXEMPLE 1

Préparation du bis {[(ditert.butyl-3,5 hydroxy-4 phényl)-3 propoxycarbonyl] -2 éthylthio} méthane.

On dissout 52,8 g, soit 0,2 mole de (ditert.butyl-3,5 hydroxy-4 phényl)-3 hydroxy-1 propane et 21,6 g (0,2 mole) d'acide mercapto-3 propanoïque dans 250 ml de toluène. Après addition de quelques gouttes d'acide sulfurique concentré, on porte le mélange réactionnel à la température de reflux du toluène. L'appareillage comporte un séparateur de type Dean-Stark et permet de recueillir l'eau formée au cours de la réaction. Après 3 heures de reflux, on recueille 3,6 ml d'eau. Une chromatographie en couche mince indique que la totalité du phénol-alcool a réagi.

On introduit alors 3,1 g (0,103 mole) de polyoxyméthylène et on porte de nouveau l'ensemble à reflux du toluène. Au bout de 2 heures de reflux on recueille 3,6 ml d'eau.

Après refroidissement, le mélange réactionnel est lavé à l'eau, décanté, et le toluène est éliminé dans un évaporateur rotatif à 80°C sous une pression de 15 mm de mercure. Le produit obtenu est un liquide sirupeux.

Analyse élémentaire

| | C | H | S |
|---|---|---|---|
| - % Calculé | 68,67 | 8,99 | 8,94 |
| - % Trouvé | 68,79 | 8,84 | 8,89 |

EXEMPLE 2

Préparation du bis {[(ditert.butyl-3,5 hydroxy-4 phényl)-2 éthoxycarbonyl] -2 éthylthio} méthane.

On dissout dans 250 ml de toluène, 50 g de (ditert.butyl-3,5 hydroxy-4 phényl)-2 éthanol-1, 21,6 g d'acide mercapto-3 propanoïque, et 3,1 g de polyoxyméthylène.

On ajoute 0,5 g d'acide p-toluènesulfonique et on porte le mélange à reflux du toluène pendant 3 heures. Un condenseur de type Dean-Stark permet de recueillir 7,2 ml d'eau. Une chromatographie en couche mince indique que la totalité de phénol-alcool a réagi.

La phase toluénique est lavée à l'eau chaude, décantée, et le toluène est éliminé à l'évaporateur rotatif à 80°C sous une pression de 15 mm de mercure.

Le produit obtenu est un liquide sirupeux.

Analyse élémentaire

| | C | H | S |
|---|---|---|---|
| - % Calculé | 67,98 | 8,78 | 9,31 |
| - % Trouvé | 68,24 | 8,55 | 9,22 |

EXEMPLE 3

Préparation du bis [(ditert. butyl-3,5 hydroxy-4 phényl)-3 propoxycarbonylméthythio] méthane.

On chauffe au reflux 52,8 g de (ditert.butyl-3,5 hydroxy-4 phényl)-3 propanol-1,18,8 g d'acide thioglycolique, 3,1 g de polyoxyméthylène, 0,5 g d'acide p-toluènesulfonique et 250 ml de toluène. Après un reflux de 3 heures, la réaction est terminée. Le produit réactionnel est lavé avec 200 ml d'eau chaude et décanté. Le toluène est éliminé à 80°C sous pression réduite. On obtient un produit liquide visqueux dont la teneur en soufre est de 9,1 % pour une valeur théorique de 9,3 %.

EXEMPLE 4

On renouvelle l'essai de l'exemple 3 en remplaçant respectivement l'acide thioglycolique et la polyoxyméthylène par les mêmes quantités molaires d'acide mercapto-3 propanoïque et d'aldéhyde benzoïque.

Dans l'α,α - bis {[(ditert. butyl-3,5 hydroxy-4 phényl)-3 propoxycarbonyl ]-2 éthylthio} toluène obtenu on trouve comme dans l'exemple 3 98 % de soufre théorique.

EXEMPLE 5

On opère de la même façon que dans l'exemple 4, mais avec 0,103 mole de cyclohéxanone à la place du benzaldéhyde.

Dans le bis{[(ditert.butyl-3,5 hydroxy-4 phényl)-3 propoxycarbonyl]-2 éthylthio} -1,1 cyclohexane obtenu on trouve 98 % de soufre théorique.

EXEMPLE 6

Dans le mode opératoire de l'exemple 4, on a remplacé le benzaldéhyde par 0,103 mole d'acétone. Ainsi dans le produit obtenu les $R^1$ et $R^2$ de la formule (I) sont des méthyles. On trouve 98,5 % du soufre théorique dans le bis{[(ditert.butyl-3,5 hydroxy-4 phényl)-3 propoxycarbonyl] -2 éthylthio }-2,2 propane obtenu.

Application des produits en tant que stabilisants.

Stabilisants du polypropylène contre les dégradations thermooxydatives.

On forme des mélanges homogènes à partir des stabilisants indiqués dans le tableau ci-après et d'une poudre fine de polypropylène de d = 0,905 g/$cm^3$ et de melt index 5 (charge 2,16 kg à 230°C) aux concentrations de 0,10 et 0,15 % en poids de chaque stabilisant par rapport au polypropylène.

Les mélanges sont calandrés sur un mélangeur à cylindres et les produits obtenus sont utilisés pour la préparation par pressage à chaud d'éprouvettes de 0,4 mm d'épaisseur.

L'essai des stabilisants s'effectue par mesure du temps d'induction à l'oxygène, à 200°C, des éprouvettes stabilisées à l'aide des produits de l'invention. On utilise pour cela un analyseur thermique différentiel qui permet l'enregistrement des variations d'enthalpie et notamment celles qui correspondent au début de dégradation du polypropylène.

Les stabilisants selon l'invention sont les composés des exemples 1 à 6. On a utilisé comme stabilisant témoin, un mélange de tétrakis (ditert.butyl-3,5 hydroxy-4 phényl)-3 proprionyloxyméthyl méthane (composé A) et de distéarylthiodipropionate (composé B) à des teneurs respectives de 0,1 et 0,2 %.

Les résultats des essais sont rapportés dans le tableau ci-après.

La supériorité des composés des exemples 1 à 6 selon l'invention sur ceux de la technique antérieure est bien nette.

| EXEMPLES<br>COMPOSES<br>(% en poids par rapport au polypropylène) | Exemple comparatif | EXEMPLES SELON L'INVENTION | | | | | | | | | | | |
|---|---|---|---|---|---|---|---|---|---|---|---|---|---|
| | 7 | 8 | 9 | 10 | 11 | 12 | 13 | 14 | 15 | 16 | 17 | 18 | 19 |
| Composé A<br>Composé B | 0,10<br>0,20 | | | | | | | | | | | | |
| Composé de l'exemple 1 | | 0,10 | 0,15 | | | | | | | | | | |
| Composé de l'exemple 2 | | | | 0,10 | 0,15 | | | | | | | | |
| Composé de l'exemple 3 | | | | | | 0,10 | 0,15 | | | | | | |
| Composé de l'exemple 4 | | | | | | | | 0,10 | 0,15 | | | | |
| Composé de l'exemple 5 | | | | | | | | | | 0,10 | 0,15 | | |
| Composé de l'exemple 6 | | | | | | | | | | | | 0,10 | 0,15 |
| Temps d'induction (en minutes) | 11 | 37 | 53 | 32 | 53 | 32 | 49 | 29 | 49 | 16 | 24 | 22 | 32 |

## Revendications

1. Composés organiques soufrés renfermant à la fois des groupes esters et un groupement dithioacétal, caractérisés en ce qu'ils répondent à la formule générale :

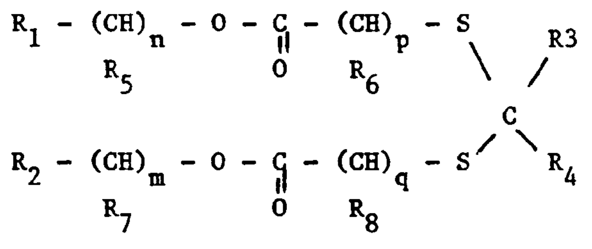

(I)

dans laquelle, $R_1$ et $R_2$, identiques ou différents, représentent des radicaux aryles, arylthio, alkylthioaryles dans lesquels le groupement aryle peut être un radical phényle substitué ou non par des hydroxyles et/ou par des radicaux hydrocarbonés aliphatiques linéaires ou ramifiés et ayant jusqu'à 6 atomes de carbone ;

$R_3$ et $R_4$, identiques ou différents, représentent des atomes d'hydrogène, des radicaux hydrocarbonés aliphatiques linéaires ou ramifiés et ayant jusqu'à 6 atomes de carbone, des radicaux cycloaliphatiques, des radicaux phényles substitués ou non par des restes hydrocarbonés aliphatiques linéaires ou ramifiés et ayant jusqu'à 6 atomes de carbone ;

$R_3$ et $R_4$, pouvant également former ensemble avec le radical méthylène et éventuellement un hétéroatome un radical cyclique ou héterocyclique ayant de 5 à 12 atomes de carbone ;

$R_5$, $R_6$, $R_7$ et $R_8$, identiques on différents, représentent des atomes d'hydrogène, des radicaux hydrocarbonés aliphatiques linéaires ou ramifiés et ayant jusqu'à 6 atomes de carbone, des radicaux cycloaliphatiques, des radicaux phényles ;

n, m, p et q sont des nombres entiers, n et m pouvant être compris entre 1 et 12, p et q pouvant être compris entre 1 et 10.

2. Composés selon la revendication 1, caractérisés en ce qu'ils répondent à la formule I, dans laquelle $R_5$, $R_6$, $R_7$ et $R_8$ représentent des atomes d'hydrogène, $R_3$ et $R_4$ représentent des atomes d'hydrogène, des radicaux hydrocarbonés aliphatiques linéaires ayant de 1 à 6 atomes de carbone, des radicaux phényles ou bien forment ensemble avec le radical méthylène un groupe cycloalkylène ayant de 5 à 12 atomes de carbone et $R_1$ et $R_2$ sont des hydroxyphényles substitués dans lesquels les groupements hydroxyles sont encombrés par 1 ou 2 restes hydrocarbonés aliphatiques ramifiés ayant de 3 à 6 atomes de carbone.

3. Composés selon l'une quelconque des revendications 1 ou 2, caractérisés en qu'ils répondent à la formule I dans laquelle $R_1$ et $R_2$, identiques ou différents, représentent l'un des groupements :

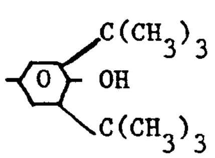 , 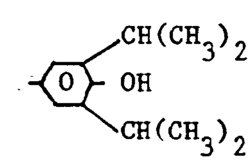 et 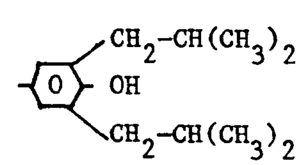

4. Le bis{[(ditert.butyl-3,5 hydroxy-4 phényl)-3 propoxycarbonyl] -2 éthylthio} méthane.

5. Le bis{[(ditert.butyl-3,5 hydroxy-4 phényl)-2 éthoxycarbonyl] -2 éthylthio} méthane.

6. Le bis [(ditert.butyl-3,5 hydroxy-4 phényl)-3 propoxycarbonylméthylthio] méthane.

7. L'α,α bis{[(ditert.butyl-3,5 hydroxy-4 phényl)-3 propoxycarbonyl] -2 éthylthio} toluène.

8. Le bis{[(ditert.butyl-3,5 hydroxy-4 phényl)-3 propoxycarbonyl] -2 éthylthio}-1,1 cyclohexane.

9. Le bis{[(ditert.butyl-3,5 hydroxy-4 phényl)-3 propoxycarbonyl] -2 éthylthio} 2,2 propane.

**10.** Procédé de préparation des composés selon l'une quelconque des revendications 1 à 9 par réaction d'un phénol-alcool, d'un mercaptoacide et d'un aldéhyde ou d'une cétone.

**11.** Procédé selon la revendication 10 caractérisé en ce qu'il consiste à faire réagir un phéno-alcool avec un mercapto-acide en présence d'un catalyseur acide puis à condenser le mercaptoester obtenu avec l'aldéhyde ou la cétone.

**12.** Procédé selon la revendication 10 caractérisé en ce qu'il consiste à condenser l'aldéhyde ou la cétone avec un mercapto-acide puis à faire réagir le dithioacétaldiacide obtenu avec un phénol-alcool en présence d'un catalyseur acide.

**13.** Procédé selon la revendication 12 caractérisé en ce que les deux réactions sont sensiblement simultanées.

**14.** Compositions de résines synthétiques caractérisées en ce qu'elles renferment au moins un composé selon l'une quelconque des revendications 1 à 9.

**15.** Compositions selon la revendication 14 caractérisées en ce que le composé est choisi dans le groupe constitué par :

- Le bis{[(ditert.butyl-3,5 hydroxy-4 phényl)-3 propoxycarbonyl] -2 éthylthio} méthane.
- Le bis{[(ditert.butyl-3,5 hydroxy-4 phényl)-2 éthoxycarbonyl] -2 éthylthio} méthane.
- Le bis [(ditert.butyl-3,5 hydroxy-4 phényl)-3 propoxycarbonylméthylthio] méthane.
- L'α,α bis{[(ditert.butyl-3,5 hydroxy-4 phényl)-3 propoxycarbonyl] -2 éthylthio} toluène.
- Le bis{[(ditert.butyl-3,5 hydroxy-4 phényl)-3 propoxycarbonyl] -2 éthylthio} -1,1 cyclohexane.
- Le bis{[(ditert.butyl-3,5 hydroxy-4 phényl)-3 propoxycarbonyl] -2 éthylthio} 2,2 propane.

## Patentansprüche

**1.** Schwefelhaltige organische Verbindungen, enthaltend gleichzeitig Estergruppen und eine Dithioacetalgruppe, gekennzeichnet durch die allgemeine Formel

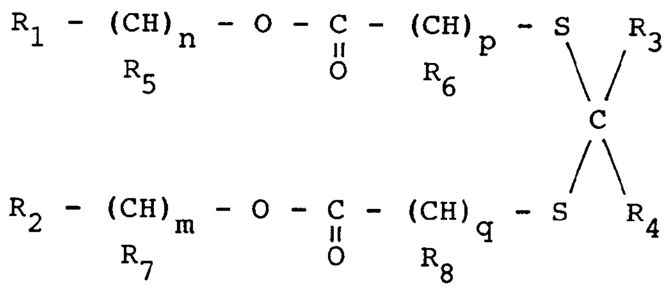

(I)

worin $R_1$ und $R_2$ gleich oder verschieden sind und Aryl-, Arylthio-, Alkylthioarylreste bedeuten, in denen die Arylgruppe ein nicht substituierter oder durch Hydroxylgruppen und/oder geradkettige oder verzweigte aliphatische Kohlenwasserstoffreste substituierter Phenylrest mit bis zu 6 Kohlenstoffatomen sein kann;
$R_3$ und $R_4$ gleich oder verschieden sind und Wasserstoffatome, geradkettige oder verzweigte, aliphatische Kohlenwasserstoffreste mit bis zu 6 Kohlenstoffatomen, cycloaliphatische Reste, nicht-substituierte oder durch geradkettige oder verzweigte aliphatische bis zu 6 Kohlenstoffatome aufweisende Kohlenwasserstoffreste substituierte Phenylreste bedeuten;
$R_3$ und $R_4$ gleichermaßen eine Einheit mit einem Methylenrest und gegebenenfalls einem Heteroatom, einem cyclischen Rest oder einem Heterocyclus mit 5 bis 12 Kohlenstoffatomen bilden können;
$R_5$, $R_6$, $R_7$ und $R_8$ gleich oder verschieden sind und Wasserstoffatome, geradkettige oder verzweigte aliphatische Kohlenwasserstoffreste mit bis zu 6 Kohlenstoffatomen, cycloaliphatische Reste und Phenylreste bedeuten;
n, m, p und q ganze Zahlen sind, wobei n und m zwischen 1 und 12 und p und q zwischen 1 und 10 betragen können.

**2.** Verbindungen nach Anspruch 1, gekennzeichnet durch die Formel I, worin $R_5$, $R_6$, $R_7$ und $R_8$ Wasserstoffatome bedeuten, $R_3$ und $R_4$ Wasserstoffatome, geradkettige aliphatische Kohlenwasserstoffreste mit 1 bis 6 Kohlenstoffatomen und Phenylreste bedeuten oder eine Einheit mit einem Methylenrest, einer Cycloalkylengruppe mit 5 bis 12 Kohlenstoffatomen bilden und $R_1$ und $R_2$ substituierte Hydroxyphenyle sind, in denen die Hydroxylgruppen durch 1 oder 2 verzweigte aliphatische, 3 bis 6 Kohlenstoffatome aufweisende Kohlenwasserstoffreste gehindert sind.

**3.** Verbindungen nach einem der Ansprüche 1 oder 2, gekennzeichnet durch die Formel I, worin $R_1$ und $R_2$ gleich oder verschieden sind und eine der Gruppen

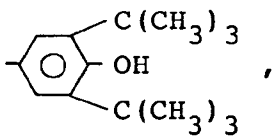 , 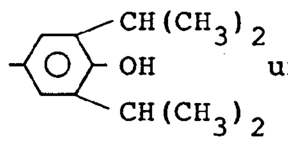 und 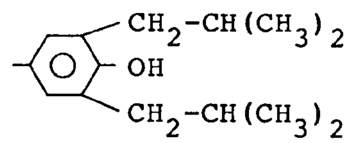

bedeuten.

**4.** Bis-{2- [3-(3,5-ditert.Butyl-4-hydroxyphenyl)-propoxycarbonyl]-ethylthio}-methan.

**5.** Bis-{2-[2-(3,5-ditert.butyl-4-hydroxyphenyl)-ethoxycarbonyl]-ethylthio}-methan.

**6.** Bis-[3-(3,5-ditert.butyl-4-hydroxyphenyl)-propoxycarbonylmethyltio]-methan.

**7.** α,α-Bis-{2-[3-(3,5-ditert.butyl-4-hydroxyphenyl)propoxycarbonyl]-methylthio}-toluol.

**8.** Bis-1,1-{2-[3-(3,5-ditert.butyl-4-hydroxyphenyl)propoxycarbonyl]-ethylthio}-cyclohexan.

**9.** Bis-2,2-{2-[3-) (3,5-ditert.butyl-4-hydroxyphenyl)propoxycarbonyl]-ethylthio}-propan.

**10.** Verfahren zur Herstellung der Verbindungen nach einem der Ansprüche 1 bis 9 durch Umsetzen eines Phenolalkohols, einer Mercaptosäure und eines Aldehyds oder eines Ketons.

**11.** Verfahren nach Anspruch 10, dadurch gekennzeichnet, daß man einen Phenolalkohol mit einer Mercaptosäure in Gegenwart eines sauren Katalysators reagieren läßt und anschließend den erhaltenen Mercaptoester mit dem Aldehyd oder dem Keton kondensiert.

**12.** Verfahren nach Anspruch 10, dadurch gekennzeichnet, daß man den Aldehyd oder das Keton mit einer Mercaptosäure kondensiert und anschließend die erhaltene Dithioacetaldisäure mit einem Phenolalkohol in Gegenwart eines sauren Katalysators reagieren läßt.

**13.** Verfahren nach Anspruch 12, dadurch gekennzeichnet, daß die zwei Reaktionen im wesentlichen gleichzeitig ablaufen.

**14.** Zusammensetzungen von synthetischen Harzen, dadurch gekennzeichnet, daß sie wenigstens eine Verbindung nach einem der Ansprüche 1 bis 9 enthalten.

**15.** Zusammensetzungen nach Anspruch 14, dadurch gekennzeichnet, daß die Verbindung aus der durch

- Bis-{2-[3-(3,5-ditert.butyl-4-hydroxyphenyl)-propoxycarbonyl]-ethylthio}-methan
- Bis-{2-{2-(3,5-ditert.butyl-4-hydroxyphenyl)-ethoxycarbonyl]-ethylthio}-methan.
- Bis-[3-(3,5-ditert.butyl-4-hydroxyphenyl)propoxycarbonylmethylthio]-methan.
- α,α -Bis{2- [3-(3,5-ditert.butyl-4-hydroxyphenyl)propoxycarbonyl]-methylthio}-toluol.
- Bis-1,1-{2-[3-(3,5-ditert.butyl-4-hydroxyphenyl)propoxycarbonyl]-ethylthio}-cyclohexan.
- Bis-2,2-{2-[3-)(3,5-ditert.Butyl-4-hydroxyphenyl)propoxycarbonyl]-ethylthio}-propan.

gebildeten Gruppe ausgewählt ist.

## Claims

1. Organic sulphur compounds containing both ester groups and a dithioacetal group, characterised in that they correspond to the general formula:

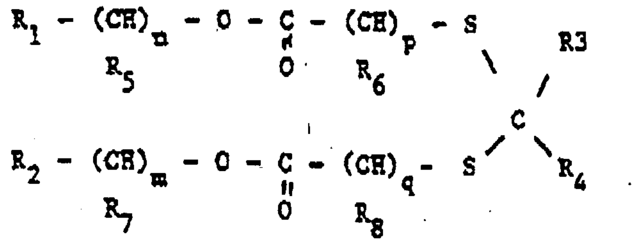

(I)

in which $R_1$ and $R_2$, which are identical or different, denote aryl, arylthio and alkylthioaryl radicals in which the aryl group may be a phenyl radical unsubstituted or substituted by hydroxyls and/or by linear or branched aliphatic hydrocarbon radicals containing up to 6 carbon atoms;

$R_3$ and $R_4$, which are identical or different, denote hydrogen atoms, linear or branched aliphatic hydrocarbon radicals containing up to 6 carbon atoms, cycloatiphatic radicals, phenyl radicals unsubstituted or substituted by linear or branched aliphatic hydrocarbon residuee containing up to 6 carbon atoms;

$R_3$ and $R_4$, together with the methylene radical and optionally a heteroatom, may also form a cyclic or heterocyclic radical containing from 5 to 12 carbon atoms;

$R_5$, $R_6$, $R_7$ and $R_6$, which are identical or different, denote hydrogen atoms, linear or branched aliphatic hydrocarbon radicals containing up to 6 carbon atoms, cycloaliphatic radicals and phenyl radicals;

n, m, p and q are integers, it being possible for n and m to be between 1 and 12, it being possible for p and q to be between 1 and 10.

2. Compounds according to Claim 1, characterised in that they correspond to the formula I, in which $R_6$, $R_6$, $R_7$ and $R_8$ denote hydrogen atoms, $R_3$ and $R_4$ denote hydrogen atoms, linear aliphatic hydrocarbon radicals containing from 1 to 6 carbon atoms, phenyl radicals, or else together with the methylene radical form a cycloalkylene group containing from 5 to 12 carbon atoms and $R_1$ and $R_2$ are substituted hydroxyphenyls in which the hydroxyl groups are hindered by one or two branched aliphatic hydrocarbon residues containing from 3 to 6 carbon atoms.

3. Compounds according to either of Claims 1 and 2, characterised in that they correspond to the formula I in which $R_1$ and $R_2$, which are identical or different, denote one of the groups:

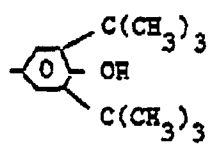
,
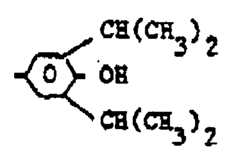
and
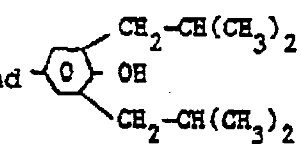

4. Bis{2-[3-(3,5-di-tert-butyl-4-hydroxyphenyl)-propoxycarbonyl]ethylthio}methane.

5. Bis{2-[2-(3,5-di-tert-butyl-4-hydroxyphenyl)ethoxycarbonyl]ethylthio}methane.

6. Bis[3-(3,5-di-tert-butyl-4-hydroxyphenyl)propoxycarbonylmethylthio]methane.

7. α,α-Bis{2-[3-(3,5-di-tert-butyl-4-hydroxyphenyl)-propoxycarbonyl]ethylthio}toluene.

8. 1,1-Bis{2-[3-(3,5-di-tert-butyl-4-hydroxyphenyl)-propoxycarbonyl]ethylthio}cyclohexane.

9. 2,2-Bis{2-[3-(3,5-di-tert-butyl-4-hydroxyphenyl)-propoxycarbonyl]ethylthio}propane.

10. Process for the preparation of the compounds according to any one of Claims 1 to 9 by reaction of a phenyl-alcohol, a mercapto acid and an aldehyde or ketone.

**11.** Process according to Claim 10, characterised in that it consists in reacting a phenol-alcohol with a mercaptoacid in the presence of an acidic catalyst and then condensing the mercaptoester obtained with the aldehyde or ketone.

**12.** Process according to Claim 10, characterised in that it consists in condensing the aldehyde or ketone with a mercaptoacid and then reacting the dithioacetaldiacid obtained with a phenol-alcohol in the presence of an acidic catalyst.

**13.** Process according to Claim 12, characterised in that the two reactions are substantially simultaneous.

**14.** Synthetic resin compositions characterised in that they contain at least one compound according to any one of Claims 1 to 9.

**15.** Compositions according to Claim 14, characterised in that the compound is chosen from the group consisting of :
- bis{2-[3-(3,5-di-tert-butyl-4-hydroxyphenyl)-propoxycarbonyl]ethylthio}methane,
- bis{2-[2-(3,5-di-tert-butyl-4-hydroxyphenyl)-ethoxycarbonyl]ethylthio}methane,
- bis[3-(3,5-di-tert-butyl-4-hydroxyphenyl)-propoxycarbonylmethylthio]methane,
- α,α-bis{2-[3-(3,5-di-tert-butyl-4-hydroxyphenyl)propoxycarbonyl]ethylthio}toluene,
- 1,1-bis{2-[3-(3,5-di-tert-butyl-4-hydroxyphenyl)propoxycarbonyl]ethylthio}cyclohexane,
- 2,2-bis{2-[3-(3,5-di-tert-butyl-4-hydroxyphenyl)propoxycarbonyl]ethylthio}propane.